# EUROPEAN PATENT SPECIFICATION

(11) **EP 4 171 355 B1**
(45) Date of publication and mention of the grant of the patent: **14.05.2025**
(21) Application number: 21733462.2
(22) Date of filing: 21.06.2021
(51) Int. Cl.: A61B 5/00, A61B 5/0205, A61B 5/026, A61B 5/08, A61B 5/11, A61B 5/113, A61B 5/055

(54) **CONTACTLESS TEMPERATURE-BASED MONITORING OF A PATIENT**
BERÜHRUNGSLOSE TEMPERATURBASIERTE ÜBERWACHUNG EINES PATIENTEN
SURVEILLANCE SANS CONTACT BASÉE SUR LA TEMPÉRATURE D'UN PATIENT

(30) Priority: 26.06.2020 EP 20182416
(43) Date of publication of application: 03.05.2023
(73) Proprietor: Koninklijke Philips N.V., 5656 AG Eindhoven (NL)
(72) Inventor: KRUEGER, Sascha, 5656 AE Eindhoven (NL)
(74) Representative: Philips Intellectual Property & Standards
(86) International application number: PCT/EP2021/066772
(87) International publication number: WO 2021/259835

(56) References cited:
- US-A1- 2014 275 832
- US-A1- 2015 157 270
- US-A1- 2019 101 655
- US-A1- 2020 155 040
- YOUNGJUN CHO ET AL: "Physiological and Affective Computing through Thermal Imaging: A Survey", ARXIV.ORG, CORNELL UNIVERSITY LIBRARY, 201 OLIN LIBRARY CORNELL UNIVERSITY ITHACA, NY 14853, 27 August 2019 (2019-08-27), XP081470670

## Description

### FIELD OF THE INVENTION

The invention relates to the field of detecting breathing motion and cardiac motion of a patient and in particular to the detection of breathing motion and cardiac motion during medical imaging examinations or scan acquisitions.

### BACKGROUND OF THE INVENTION

The detection of breathing motion and cardiac motion of a patient has conventionally been done using sensors applied to the patient. Physiology signals for breathing and pulse may be provided by the analysis of a live video stream of a patient either in RGB or IR mode. For detection of breathing motion, visibility of the motion of the target body part such as the chest or the upper abdomen is required. In typical medical imaging scenarios coils, accessories, blankets or support devices may obstruct the view from a particular fixed view angle thus limiting the applicability of this technique. For non-obstructive remote cardiac physiology signal detection, also video data can be used to detect the color changes in the skin due to the pulsatile flow. The principle of measurement is then based on the pulse plethysmography principle. The signal strength may be reduced by different aspects, i.e. by the visibility of the skin due to top or facial hair or by the physiologic delay which may vary from patient to patient or for a single patient even over time. In general, video-based pulse plethysmography works well with a multi-spectral detector so that absorption and reflection properties of the skin can be self-calibrated for each video-frame. A preferred setup for such a technique is based on a visible RGB camera employing visible white light.

In Hu M, Zhai G, Li D, Fan Y, Duan H, Zhu W, et al. "Combination of near-infrared and thermal imaging techniques for the remote and simultaneous measurements of breathing and heart rates under sleep situation" in PLoS ONE 13(1) 2018 a far-infrared imager and an infrared camera equipped with IR-Cut lens and an infrared lighting array are described. The heart rate is detected by the infrared imaging system based on the absorption changes of the skin due to the blood flow in order to describe the quality of sleep.

The US patent application US2014/275832 discloses a device for obtaining vital signs information of an object (patient to be examined). The known device includes for example an infrared camera to acquire an image data set of the patient's skin portion. Vital sign information may then be derived from colour changes of the skin portion.

The paper from Y. Cho and N. Bianchi-Berthouze "Physiological and Affective Computing through Thermal Imaging: A Survey" concerns imaging based monitoring of bodily functions. The thermal imaging is based in natural emission of electro-magnetic (EM)radiation from (sub-)cutaneous skin region, notably of facial regions. The imaging relates to temperature changes due to vasoconstriction and vasodilation. These temperature changes are associated with the heat transfer due to blood flow, cardiac pulse rate may thereby be derived.

### SUMMARY OF THE INVENTION

It is an object of the invention to provide a monitoring method and a monitoring system for detecting at least cardiac motion of a patient in an easy, versatile and reliable way, especially without the need of irradiating additional light.

According to the invention, this object is addressed by the subject matter of the independent claims. Preferred embodiments of the invention are described in the sub claims.

Therefore, according to the invention, a method for detecting at least one physiological signal of a patient is provided, wherein the method comprises the following method steps: monitoring at least a subsection of a patient's surface with a thermal camera which generates consecutive video frames with multiple pixels of the monitored subsection, wherein the subsection of a patient's surface includes at least a part of the mouth (including the lips) and/or nose area of the patient as a region of interest; generating time-resolved temperature values of at least one pixel of the region of interest; and generating a cardiac signal as the physiological signal based on the generated time-resolved temperature values due to temperature changes due to breathing airflow.

If a cardiac signal is mentioned, a signal generated from the vibration wave following the contraction of the heart muscle in the chest is meant. The heart rate shown by the cardiac signal is the speed of the heartbeat which is typically measured by the number of heart beats per minute.

The method is adapted for generating a physiological signal based on temperature changes due to breathing air flow. That is why the region of interest includes the mouth and nose area or only the mouth or only the nose area.

The method concerns to generate a physiological signal based on temperature changes due to breathing air flow. This is done using a region of interest that includes the mouth and nose area or only the mouth or only the nose area. The thermal camera has a sensitivity range for wavelengths in the range of of 2-25µm, i.e. well beyond the near-infrared and optical wavelength ranges. An insight of the invention is that nose and mouth have areas with mucous membranes which are typically more humid than normal skin. Airflow drastically changes the temperature of this surfaces resulting in a high SNR signal, representing both respiratory and cardiac activity. The respiratory signal is directly related to inspiration and expiration air flow. When the patient inhales the mucous surface is cooled and the temperature drops significantly and rises again on exhalation of warm air from inside generating the temporal signal.

According to a further aspect of the invention, the nose and mouth areas of the patient to be examined may be easily automatically recognised for example from the thermal video frames. The automatic recognition of the nose/and mouth area of may be done with simple commercially available face recognition software, which may be implemented on the basis of deep learning as often installed nowadays on mobile telephones with an integrated camera.

A broader insight of the present invention is that acquiring long wavelength infrared data from the nose and mouth area enables to derive accurate information on the patient cardiac and respiratory motion from the temperature variations represented in the long wavelength infrared data. In addition, workflow is made more efficient because the nose and mouth area are easily and accurately automatically identified or recognised from thermal or optical video frames.

According to a preferred embodiment of the invention, the method further comprises the method step of generating a respiration signal as an additional physiological signal based on the generated time-resolved temperature values.

If a respiration signal is mentioned, a signal generated from the inhalation and exhalation process of a patient's breathing is meant. The breathing rate is the number of breaths a person takes per time, e.g. per minute. During generating a respiration signal based on the generated temperature values, a low pixel temperature relative to the reference temperature indicates inhalation and a high pixel temperature relative to the reference temperature indicates exhalation.

According to a preferred embodiment of the invention, the method further comprises the method step of triggering and/or gating a scan acquisition based on the at least one physiological signal, wherein the scan acquisition is carried out during medical imaging examination with a medical imaging device and/or during medical therapy. Breathing and heart motions cause uncertainties during medical imaging examinations and/or during medical therapies, because organs, or ROIs in general, are moving during scan acquisition. The image data acquisition is triggered in dependence on the breathing motion and/or the cardiac motion, so that the uncertainties because of moving ROIs during scan acquisition are reduced. A medical imaging device can be for example MRI, CT, PET and a medical therapy can be for example radiotherapy.

A physiological signal is generated based on temperature changes due to breathing air flow. For generating temperature value variations due to breathing air flow, the region of interest includes the mouth and nose area or only the mouth or only the nose area. According to a preferred embodiment of the invention, the at least one pixel is from the pixels covering the patient's nostrils.

One advantage resides in providing a method, wherein in the step of generating time-resolved temperature values of at least one pixel of the region of interest one single pixel is used. Hence, it is not necessary to generate time-resolved values of all pixels of the region of interest. Time-resolved temperature values of only one single pixel may be sufficient to generate a physiological signal.

Further, according to the invention, a monitoring system for detecting at least one physiological signal of a patient is provided. The monitoring system comprises a thermal camera which is adapted for monitoring at least a subsection of a patient's surface. The thermal camera generates consecutive video frames with multiple pixels of the monitored subsection. The subsection of the patient's surface includes at least a part of the mouth and/or nose area of the patient as a region of interest. The monitoring system further comprises a signal processing unit which is adapted for generating time-resolved temperature values of at least one pixel of the region of interest and for generating a cardiac signal as the physiological signal based on the generated time-resolved temperature values due to temperature changes due to breathing airflow.

According to a preferred embodiment of the invention, the monitoring system comprises a patient support which is adapted for holding the patient in such a way that the subsection of the patient's surface which may be monitored by the thermal camera comprises at least a part of the mouth and/or nose area of the patient. The patient support may be designed as a patient table that may be movable in all three spatial directions so that the positioning of the patient can be done very precisely. Additionally, also face and/or body tracking devices and algorithms can be used to further confine the region of interest. The camera itself may be such a device and known computer vision algorithms could be applied to determine e.g. facial or head area.

According to a preferred embodiment of the invention, the signal processing unit is adapted for generating a respiration signal based on the generated time-resolved temperature values as an additional physiological signal. For generating time-resolved temperature values, preferably a frequency range for the pulse is defined. The range may be for example between 40 and 300 beats per minute. For generating a second physiologically signal, preferably a range for respiration is defined. The range may be for example 10 to 20 breathing cycles per minute. By defining ranges, the contributions by band-passing may be separated. According to another preferred embodiment it is made use of the spatial shifting of the head due to the respiration which is larger than the pulse-induced motion. This shifting may be measured in order to get a clean respiration curve and to use its frequency to separate it in the thermal signal.

Further, according to the invention, a non-transitory computer-readable medium is provided which comprises instructions stored thereon, that when executed on a processor, induce a monitoring system with a thermal camera to perform the method a method for detecting at least one physiological signal of a patient.

### BRIEF DESCRIPTION OF THE DRAWINGS

These and other aspects of the invention will be apparent from and elucidated with reference to the embodiments described hereinafter. Such an embodiment does not necessarily represent the full scope of the invention, however, and reference is made therefore to the claims and herein for interpreting the scope of the invention.

In the drawings:
Fig. 1 schematically depicts a scheme of a method according to a preferred embodiment of the invention;
Fig. 2 schematically depicts a second scheme of a method according to a preferred embodiment of the invention;
Fig. 3 schematically depicts a monitoring system according to a preferred embodiment of the invention; and
Fig. 4 schematically depicts a thermal camera and a ROI of a monitoring system according to a preferred embodiment of the invention.

### DETAILED DESCRIPTION OF EMBODIMENTS

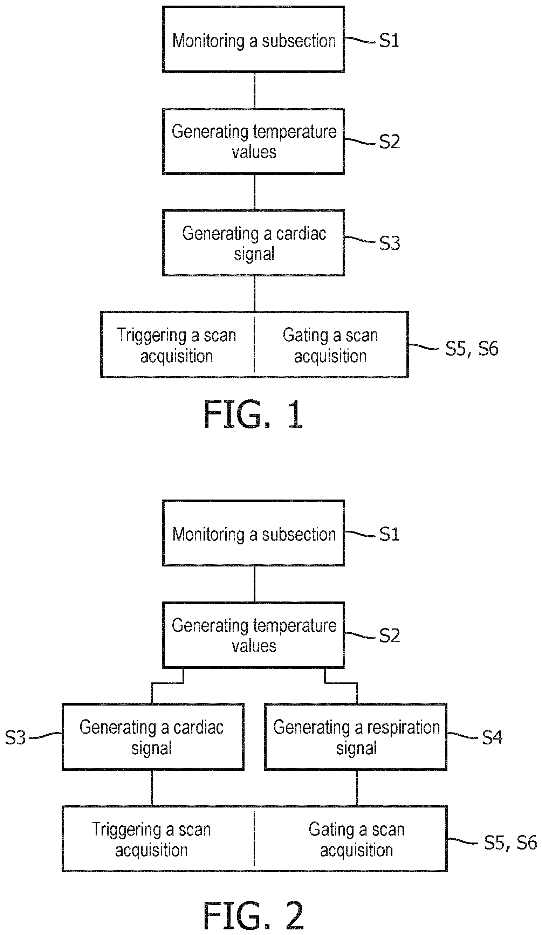

Fig. 1 schematically depicts a scheme of a method according to a preferred embodiment of the invention. The first step S1 is to monitor at least a subsection 2 of a patient's surface 4 with a thermal camera 5. The thermal camera 5 generates consecutive video frames with multiple pixels 6 of the monitored subsection 2. In order to generate a physiological signal based on temperature changes due to breathing air flow, the subsection 2 of a patient's surface 4 includes at least a part of the mouth and/or nose area of the patient 7 as a region of interest 3. The second step S2 is to generate these time-resolved temperature values of at least one pixel 8 of the region of interest 3. The third step S3 is to generate a cardiac signal 9 as the physiological signal 1 based on the generated time-resolved temperature values. The generated cardiac signal 9 can be used for the fifth step S5 and/or for the sixth step S6. The fifth step S5 is to trigger and the sixth step S6 is to gate a scan acquisition based on the at least one physiological signal 1.

A scan acquisition is carried out during medical imaging examination with a medical imaging device 11 and/or during medical therapy. Hence, it is possible to reduce uncertainties because of heart motions during medical imaging examinations and/or during medical therapies. Some regions are moving during scan acquisition because of the beating heart. The image data acquisition may be gated in dependence on the cardiac signal 9, so that the cardiac motion can be traced on medical images. Another opportunity is to trigger the image data acquisition in dependence on the cardiac signal 9, so that the image data acquisition is triggered every time the heart is in the same position. In Fig. 1 the medical imaging device 11 is a MRI system, a CT or a linear accelerator.

Fig. 2 schematically depicts a second scheme of a method according to a preferred embodiment of the invention. After the second step S2, thus after generating time-resolved temperature values of at least one pixel 8 of the region of interest 3, it is not only possible to generate a cardiac signal, as it is described in the third step S3 in Fig. 1, it is also possible to generate a respiration signal 10 as an additional physiological signal 1 based on the generated time-resolved temperature values. Generating a respiration signal 10 is the fourth step S4. Based on the cardiac signal 9 and/or the respiration signal 10 the image data acquisition may be triggered S5 and/or gated S6.

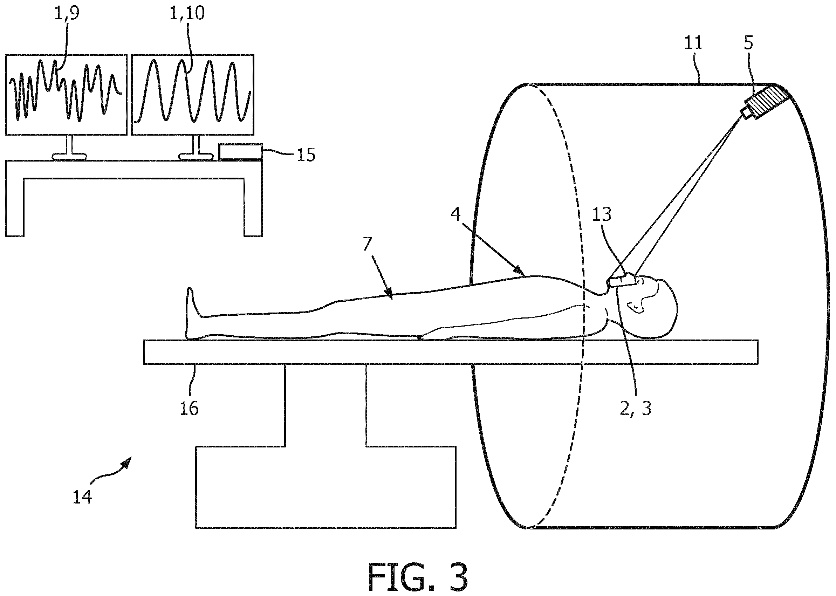

Fig. 3 schematically depicts a monitoring system 14 according to a preferred embodiment of the invention. A situation during image data acquisition is shown. A patient 7 is lying on a patient table 16. The table is movable in all three dimensions so that the region of interest 3 may be positioned precisely. The region of interest 3 is a part of a subsection 2 of the patient 7 and includes the patient's nostrils 13. A thermal camera 5 is attached to the medical imaging device 11. In Fig. 3 the medical imaging device 11 is a MRI. The thermal camera 5 scans the patient's surface 4, especially the precisely positioned region of interest 3. The thermal camera 5 is adapted to generate time-resolved temperature-values that are processed by a signal processing unit 15. The signal processing unit 15 generates two physiological signals based on the time-resolved temperature-values: a cardiac signal 9 and a respiration signal 10 that are visualized.

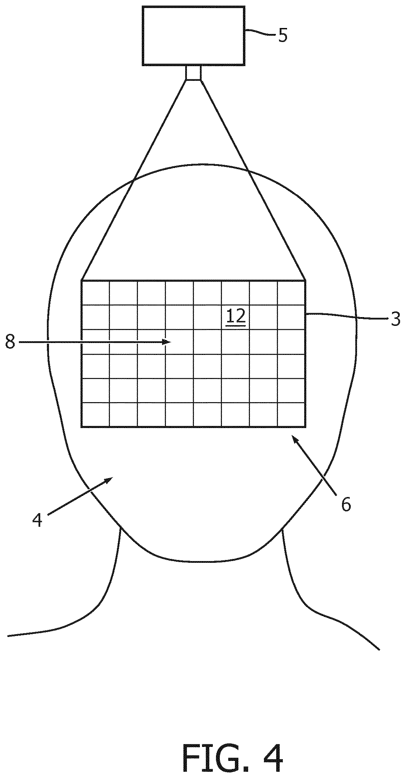

Fig. 4 schematically depicts a thermal camera 5 and a region of interest 3 of a monitoring system according to a preferred embodiment of the invention. The thermal camera 5 scans the patient's surface 4 and generates consecutive video frames with multiple pixels 6 of the region of interest 3. For the sake of clarity, the patient's nostrils 13 are not shown in Fig. 4, but they are part of the region of interest 3. Time-resolved temperature values of at least one pixel 8 of the region of interest 3 are generated. Especially according to the invention, it is possible to generate time-resolved temperature values of at least one pixel 8 of the region of interest 3 with only one single pixel 12 used.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope. Further, for the sake of clearness, not all elements in the drawings may have been supplied with reference signs. The invention is defined by the appended claims.

### REFERENCE SYMBOL LIST

| | |
|---|---|
| monitoring a subsection | S1 |
| generating temperature values | S2 |
| generating a cardiac signal | S3 |
| generating respiration signal | S4 |
| triggering a scan acquisition | S5 |
| gating a scan acquisition | S6 |
| physiological signal | 1 |
| subsection | 2 |
| region of interest | 3 |
| patient's surface | 4 |
| thermal camera | 5 |
| multiple pixels | 6 |
| patient | 7 |
| pixel | 8 |
| cardiac signal | 9 |
| respiration signal | 10 |
| medical imaging device | 11 |
| one single pixel | 12 |
| nostrils | 13 |
| monitoring system | 14 |
| signal processing unit | 15 |
| patient table | 16 |

## Claims

1. A method for detecting at least one physiological signal (1) of a patient (7), wherein the method comprises the following method steps:
monitoring at least a subsection (2) of a patient's surface (4) with a thermal camera (5) which generates consecutive video frames with multiple pixels (6) of the monitored subsection (2), wherein the subsection (2) of a patient's surface (4) includes at least a part of a humid portion of the surface, in particular the mouth and/or nose area of the patient (7) as a region of interest (3);
generating time-resolved temperature values of at least one pixel (8) of the region of interest (3); and
generating a cardiac signal (9) as the physiological signal (1) based on the generated time-resolved temperature values due to temperature changes due to breathing air flow.

2. The method of claim 1, wherein the camera detects the video frames in the wavelength region of 2µm to 25µm.

3. The method of Claim 1 or 2, the patient's nose and mouth are automatically detected as the region-of-interest from the video frames.

4. The method of any one of the preceding Claims, wherein the method comprises the method step of
generating a respiration signal (10) as an additional physiological signal (1) based on the generated time-resolved temperature values.

5. The method of any one of the preceding Claims, wherein the method comprises the method step of
triggering and/or gating a scan acquisition based on the at least one physiological signal (1), wherein the scan acquisition is carried out during medical imaging examination with a medical imaging device (11) and/or during medical therapy.

6. The method of any of the previous claims, wherein the at least one pixel (12) is from the pixels (6) covering the patient's nostrils (13).

7. The method of any of the previous claims, wherein in the step of generating time-resolved temperature values of at least one pixel (8) of the region of interest (3) one single pixel (12) is used.

8. A monitoring system (14) for detecting at least one physiological signal (1) of a patient (7), wherein the monitoring system (14) comprises
a thermal camera (5) which is adapted for monitoring at least a subsection (2) of a patient's surface (4) generating consecutive video frames with multiple pixels (6) of the monitored subsection (2), wherein the subsection (2) of the patient's surface (4) includes at least a humid portion of the surface, in particular part of the mouth and/or nose area of the patient (7) as a region of interest (3); and
a signal processing unit (15) which is adapted for generating time-resolved temperature values of at least one pixel (8) of the region of interest (3) and for generating a cardiac signal (9) as the physiological signal (1) based on the generated time-resolved temperature values due to temperature changes due to breathing air flow

9. The monitoring system (14) of Claim 8, wherein the thermal camera is sensitive in an infrared wavelength range of 2µm to 25 µm.

10. The monitoring system (14) of anyone of claims 8 or 9, wherein the signal processing unit is provided with an automatic detection module to identify the patient's nose and mouth area from the video frames.

11. The monitoring system (14) of claim 8 or 9, wherein the signal processing unit (15) is adapted for generating a respiration signal (10) based on the generated time-resolved temperature values as an additional physiological signal (1).

12. A non-transitory computer-readable medium, comprising instructions stored thereon, that when executed on a processor, induce a monitoring system (14) with a thermal camera (5) to perform the method according to any of Claims 1 to 7.

## Patentansprüche

1. Verfahren zum Erfassen mindestens eines physiologischen Signals (1) eines Patienten (7), wobei das Verfahren die folgenden Verfahrensschritte umfasst:
Überwachen mindestens eines Teilabschnitts (2) einer Patientenoberfläche (4) mit einer Wärmekamera (5), die aufeinanderfolgende Videobilder mit multiplen Pixeln (6) des überwachten Teilabschnitts (2) erzeugt, wobei der Teilabschnitt (2) einer Patientenoberfläche (4) mindestens einen Teil eines feuchten Abschnitts der Oberfläche, insbesondere den Mund- und/oder Nasenbereich des Patienten (7) als Region von Interesse (3) einschließt;
Erzeugen zeitaufgelöster Temperaturwerte von mindestens einem Pixel (8) der Region von Interesse (3); und
Erzeugen eines Herzsignals (9) als das physiologische Signal (1) basierend auf den erzeugten zeitaufgelösten Temperaturwerten aufgrund von Temperaturänderungen aufgrund des Atemluftstroms.

2. Verfahren nach Anspruch 1, wobei die Kamera die Videobilder in der Wellenlängenregion von 2 µm bis 25 µm erfasst.

3. Verfahren nach Anspruch 1 oder 2, wobei die Nase und der Mund des Patienten aus den Videobildern automatisch als die Region von Interesse erfasst werden.

4. Verfahren nach einem der vorstehenden Ansprüche, wobei das Verfahren den Verfahrensschritt des
Erzeugens eines Atmungssignals (10) basierend auf den erzeugten zeitaufgelösten Temperaturwerten als ein zusätzliches physiologisches Signal (1) umfasst.

5. Verfahren nach einem der vorstehenden Ansprüche, wobei das Verfahren den Verfahrensschritt des
Auslösens und/oder Ansteuerns einer Scan-Aufnahme basierend auf dem mindestens einen physiologischen Signal (1) umfasst, wobei die Scan-Aufnahme im Laufe einer medizinischen Bildgebungsuntersuchung mit einer medizinischen Bildgebungsvorrichtung (11) und/oder im Laufe der medizinischen Therapie ausgeführt wird.

6. Verfahren nach einem der vorstehenden Ansprüche, wobei das mindestens eine Pixel (12) von den Pixeln (6) stammt, die die Nasenlöcher (13) des Patienten abdecken.

7. Verfahren nach einem der vorstehenden Ansprüche, wobei in dem Schritt des Erzeugens zeitaufgelöster Temperaturwerte von mindestens einem Pixel (8) der Region von Interesse (3) ein einzelnes Pixel (12) verwendet wird.

8. Überwachungssystem (14) zum Erfassen mindestens eines physiologischen Signals (1) eines Patienten (7), wobei das Überwachungssystem (14) umfasst
eine Wärmekamera (5), die zum Überwachen mindestens eines Teilabschnitts (2) einer Patientenoberfläche (4) angepasst ist, die aufeinanderfolgende Videobilder mit multiplen Pixeln (6) des überwachten Teilabschnitts (2) erzeugt, wobei der Teilabschnitt (2) der Patientenoberfläche (4) mindestens einen feuchten Abschnitt der Oberfläche, insbesondere einen Teil des Mund- und/oder Nasenbereichs des Patienten (7) als Region von Interesse (3) einschließt; und
eine Signalverarbeitungseinheit (15), die zum Erzeugen zeitaufgelöster Temperaturwerte von mindestens einem Pixel (8) der Region von Interesse (3) und zum Erzeugen eines Herzsignals (9) als das physiologische Signal (1) basierend auf den erzeugten zeitaufgelösten Temperaturwerten aufgrund von Temperaturänderungen aufgrund des Atemluftstroms angepasst ist.

9. Überwachungssystem (14) nach Anspruch 8, wobei die Wärmekamera in einem Infrarot-Wellenlängenbereich von 2 µm bis 25 µm empfindlich ist.

10. Überwachungssystem (14) nach einem der Ansprüche 8 oder 9, wobei die Signalverarbeitungseinheit mit einem automatischen Erfassungsmodul bereitgestellt ist, um den Nasen- und Mundbereich des Patienten aus den Videobildern zu identifizieren.

11. Überwachungssystem (14) nach Anspruch 8 oder 9, wobei die Signalverarbeitungseinheit (15) zum Erzeugen eines Atmungssignals (10) basierend auf den erzeugten zeitaufgelösten Temperaturwerten als ein zusätzliches physiologisches Signal (1) angepasst ist.

12. Nichtflüchtiges computerlesbares Medium, das darauf gespeicherte Anweisungen umfasst, die, wenn sie auf einem Prozessor ausgeführt werden, ein Überwachungssystem (14) mit einer Wärmekamera (5) veranlassen, das Verfahren nach einem der Ansprüche 1 bis 7 durchzuführen.

## Revendications

1. Procédé de détection d'au moins un signal physiologique (1) d'un patient (7), dans lequel le procédé comprend les étapes de procédé suivantes consistant à :
surveiller au moins une sous-section (2) de la surface d'un patient (4) avec une caméra thermique (5) qui génère des images vidéo consécutives avec de multiples pixels (6) de la sous-section surveillée (2), dans lequel la sous-section (2) de la surface d'un patient (4) inclut au moins une partie d'une portion humide de la surface, en particulier la zone de la bouche et/ou du nez du patient (7), à titre de région d'intérêt (3) ;
générer des valeurs de température à résolution temporelle d'au moins un pixel (8) de la région d'intérêt (3) ; et
générer un signal cardiaque (9) au titre du signal physiologique (1) sur la base des valeurs de température à résolution temporelle générées en raison de variations de température liées au flux d'air respiratoire.

2. Procédé selon la revendication 1, dans lequel la caméra détecte les images vidéo dans la région de longueurs d'ondes de 2 µm à 25 µm.

3. Procédé selon la revendication 1 ou 2, dans lequel le nez et la bouche du patient sont détectés automatiquement au titre de la région d'intérêt à partir des images vidéo.

4. Procédé selon l'une quelconque des revendications précédentes, dans lequel le procédé comprend l'étape de procédé consistant à
générer un signal de respiration (10) à titre de signal physiologique supplémentaire (1) sur la base des valeurs de température à résolution temporelle générées.

5. Procédé selon l'une quelconque des revendications précédentes, dans lequel le procédé comprend l'étape de procédé consistant à
déclencher et/ou synchroniser une acquisition par balayage sur la base de l'au moins un signal physiologique (1), dans lequel l'acquisition par balayage est réalisée pendant un examen d'imagerie médicale avec un dispositif d'imagerie médicale (11) et/ou pendant une thérapie médicale.

6. Procédé selon l'une quelconque des revendications précédentes, dans lequel le au moins un pixel (12) figure parmi les pixels (6) couvrant les narines (13) du patient.

7. Procédé selon l'une quelconque des revendications précédentes, dans lequel, pendant l'étape de génération de valeurs de température à résolution temporelle d'au moins un pixel (8) de la région d'intérêt (3), un seul pixel (12) est utilisé.

8. Système de surveillance (14) conçu pour détecter au moins un signal physiologique (1) d'un patient (7), dans lequel le système de surveillance (14) comprend
une caméra thermique (5) qui est adaptée pour surveiller au moins une sous-section (2) de la surface d'un patient (4) en générant des images vidéo consécutives avec de multiples pixels (6) de la sous-section surveillée (2), dans lequel la sous-section (2) de la surface du patient (4) inclut au moins une partie humide de la surface, en particulier une partie de la zone de la bouche et/ou du nez du patient (7), à titre de région d'intérêt (3) ; et
une unité de traitement de signal (15) qui est adaptée pour générer des valeurs de température à résolution temporelle d'au moins un pixel (8) de la région d'intérêt (3) et pour générer un signal cardiaque (9) au titre du signal physiologique (1) sur la base des valeurs de température à résolution temporelle générées en raison de variations de température liées au flux d'air respiratoire.

9. Système de surveillance (14) selon la revendication 8, dans lequel la caméra thermique est sensible dans une plage de longueurs d'ondes infrarouges de 2 µm à 25 µm.

10. Système de surveillance (14) selon l'une quelconque des revendications 8 ou 9, dans lequel l'unité de traitement de signal est équipée d'un module de détection automatique de façon à identifier la zone du nez et de la bouche du patient à partir des images vidéo.

11. Système de surveillance (14) selon la revendication 8 ou 9, dans lequel l'unité de traitement de signal (15) est adaptée pour générer un signal de respiration (10) sur la base des valeurs de température à résolution temporelle générées à titre de signal physiologique supplémentaire (1).

12. Support non transitoire lisible par ordinateur, comprenant des instructions stockées sur celui-ci qui, lorsqu'elles sont exécutées sur un processeur, amènent un système de surveillance (14) avec une caméra thermique (5) à exécuter le procédé selon l'une quelconque des revendications 1 à 7.
